# EUROPEAN PATENT APPLICATION

(11) **EP 4 108 296 A1**
(43) Date of publication of application: **28.12.2022**
(21) Application number: 21756870.8
(22) Date of filing: 29.01.2021
(51) Int. Cl.: A61N 5/06

(54) **PHOTOCOSMETIC DEVICE**

(30) Priority: 21.02.2020 JP 2020028766
(71) Applicant: Panasonic Intellectual Property Management Co., Ltd., Osaka-shi, Osaka 540-6207 (JP)
(72) Inventor: HIRAYAMA, Yutsuki, Osaka-shi, Osaka 540-6207 (JP); TATSUTA, Shigeru, Osaka-shi, Osaka 540-6207 (JP); KANEKO, Shota, Osaka-shi, Osaka 540-6207 (JP)
(74) Representative: Grünecker Patent- und Rechtsanwälte PartG mbB
(86) International application number: PCT/JP2021/003189
(87) International publication number: WO 2021/166590

(57) **Abstract**

A photocosmetic device according to the present disclosure includes a light source and a controller. The light source outputs illumination light which illuminates a target portion. The controller controls an output of the light source, an on-duty ratio of the light source, and a lighting frequency of the light source. A peak wavelength of the light source is included in a range from 600 nm to 980 nm inclusive. A half-spectral width of the light source is included in a range from 20 nm to 80 nm inclusive. The controller controls the light source such that the output of the light source is included in a range from 30 mW/cm² to 100 mW/cm² inclusive, the on-duty ratio of the light source is included in a range from 20% to 80% inclusive, and the lighting frequency of the light source is included in a range from 0.1 kHz to 30 kHz inclusive.

## Description

### TECHNICAL FIELD

The present disclosure relates to a photocosmetic device.

### BACKGROUND ART

A photocosmetic device includes a light source. The light source outputs light to a target portion. A photocosmetic device described in PTL 1 outputs light having peaks at 520 nm, 660 nm, and 780 nm, respectively. A photocosmetic device described in PTL 2 outputs light having peaks in ranges from 570 nm to 600 nm inclusive and from 750 nm to 850 nm inclusive.

### Citation List

### Patent Literatures

PTL 1: Unexamined Japanese Patent Publication No. 2018-507740
PTL 2: Unexamined Japanese Patent Publication No. 2002-537940

### SUMMARY OF THE INVENTION

When light illuminates a living body from the light source, there is a concern that the living body is damaged. It is expected that the photocosmetic device hardly damages the living body.

A photocosmetic device of the present disclosure includes a light source that outputs illumination light which illuminates a target portion, and a controller that controls an output of the light source, a lighting frequency of the light source, and an on-duty ratio of the light source. A peak wavelength of the light source is included in a range from 600 nm to 980 nm inclusive, and a spectral half-value width of the light source is included in a range from 20 nm to 80 nm inclusive. The controller controls the light source, the output of the light source being included in a range from 30 mW/cm² to 100 mW/cm² inclusive, the lighting frequency of the light source being included in a range from 0.1 kHz to 30 kHz inclusive, and the on-duty ratio of the light source being included in a range from 20% to 80% inclusive.

The photocosmetic device of the present disclosure hardly damages the living body.

### BRIEF DESCRIPTION OF THE DRAWINGS

Fig. 1 is a diagram illustrating a model of a photocosmetic device.
Fig. 2 is a diagram illustrating a table showing conditions and results of tests.
Fig. 3 is a diagram illustrating a table showing conditions and results of tests.
Fig. 4 is a diagram illustrating a table showing conditions and results of tests.
Fig. 5 is a diagram illustrating a table showing conditions and results of tests.
Fig. 6 is a diagram illustrating a table showing conditions and results of tests.

### DESCRIPTION OF EMBODIMENT

### (Exemplary modes of photocosmetic device)

A photocosmetic device includes a light source that outputs illumination light which illuminates a target portion, and a controller that controls an output of the light source, a lighting frequency of the light source, and an on-duty ratio of the light source. A peak wavelength of the light source is included in a range from 600 nm to 980 nm inclusive, a spectral half-value width of the light source is included in a range from 20 nm to 80 nm inclusive, and the controller controls the light source, the output of the light source being included in a range from 30 mW/cm² to 100 mW/cm² inclusive, the lighting frequency of the light source being included in a range from 0.1 kHz to 30 kHz inclusive, and the on-duty ratio of the light source being included in a range from 20% to 80% inclusive.

According to the photocosmetic device, damage of a living body is suppressed, and an effect of improving a state of the living body is enhanced.

In an example of the photocosmetic device, an on-duty ratio of the light source is included in a range from 40% to 80% inclusive.

According to the photocosmetic device, a more preferable effect can be obtained.

In an example of the photocosmetic device, an on-duty ratio of the light source is included in a range from 40% to 60% inclusive.

According to the photocosmetic device, a more preferable effect can be obtained.

In an example of the photocosmetic device, a peak wavelength of the light source is included in a range from 820 nm to 980 nm inclusive.

According to the photocosmetic device, a more preferable effect can be obtained.

In an example of the photocosmetic device, a peak wavelength of the light source is included in a range from 900 nm to 980 nm inclusive.

According to the photocosmetic device, a more preferable effect can be obtained.

In an example of the photocosmetic device, a lighting frequency of the light source is included in a range from 10 kHz to 30 kHz inclusive.

According to the photocosmetic device, a more preferable effect can be obtained.

### (Exemplary embodiment)

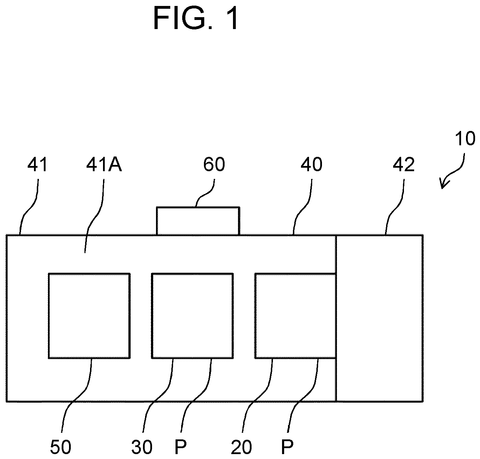

As illustrated in Fig. 1, photocosmetic device 10 includes light source 20. A mode of photocosmetic device 10 is, for example, a portable type or a stationary type. Light source 20 outputs illumination light which illuminates a target portion. The target portion is, for example, human skin. The illumination light may have a preferable cosmetic action on the target portion.

Light source 20 includes, for example, a light emitting diode (LED). In one example, light source 20 includes a surface-mounted LED and a board. The LED is, for example, a GaAs-based LED. The LED is mounted on a board. In one example, light source 20 includes a plurality of LEDs. The plurality of LEDs are electrically connected. A mode of the connection is selected from, for example, a series connection, a parallel connection, and a combined connection of a series connection and a parallel connection.

Photocosmetic device 10 further includes controller 30. Controller 30 is electrically connected to light source 20. Controller 30 includes, for example, a processor. Controller 30 controls light source 20. In one example, controller 30 controls power input to light source 20 by pulse width modulation (PWM) control.

In one example, photocosmetic device 10 further includes housing 40, power supply 50, and switch 60. Housing 40 constitutes an outer shell of photocosmetic device 10. Housing 40 includes case 41 and cover 42. Case 41 includes inner space 41A in which various elements constituting photocosmetic device 10 can be disposed. Case 41 is, for example, a resin molded article. Examples of a material of case 41 include polycarbonate. Cover 42 is attached to case 41. Cover 42 protects light source 20. Illumination light of light source 20 is transmitted through cover 42. Cover 42 is made of a transparent material. In one example, cover 42 is made of a near-infrared transmissive material. Examples of the near-infrared transmissive material include quartz glass and acrylic resin.

Power supply 50 is provided in housing 40. In one example, power supply 50 is provided in inner space 41A of case 41. Power supply 50 is electrically connected to power demand unit P included in photocosmetic device 10. Power supply 50 supplies power of a primary battery, a secondary battery, or an external power supply to power demand units P. Examples of power demand units P are light source 20 and controller 30. An example of the external power supply is a commercial AC power supply.

Switch 60 is provided in case 41. Switch 60 is configured to be operable by a user. An operation state of switch 60 is switched according to an operation of switch 60. When the operation state of switch 60 is an off-state, the power of power supply 50 is not supplied to power demand units P. When the operation state of switch 60 is an on-state, the power of power supply 50 is supplied to power demand units P. Switch 60 is electrically connected to controller 30.

Features of light source 20 will be described. The features of light source 20 include, for example, the number of peaks of light source 20, a peak wavelength of light source 20, a spectral half-value width of light source 20, and a half-value angle of light source 20. The number of peaks of light source 20 is the number of peaks in the spectrum of the illumination light. The peak wavelength of light source 20 is a peak wavelength in the spectrum of the illumination light. The spectral half-value width of light source 20 is a peak half-value width in the spectrum of the illumination light.

The number of peaks of light source 20 will be exemplified. In a first example, light source 20 has a single peak. In a second example, light source 20 has a plurality of peaks. The peak wavelength of light source 20 will be exemplified. In a first example, the peak wavelength is included in a range from 600 nm to 980 nm inclusive. In a second example, the peak wavelength is included in a range from 820 nm to 980 nm inclusive. In a third example, the peak wavelength is included in a range from 900 nm to 980 nm inclusive. The peak wavelength in the example in which light source 20 includes the plurality of peaks is a peak wavelength corresponding to a maximum output value in the spectrum of the illumination light. The spectral half-value width of light source 20 will be exemplified. In a first example, the spectral half-value width is included in a range from 20 nm to 80 nm inclusive. In a second example, the spectral half-value width is included in a range from 40 nm to 80 nm inclusive. The half-value angle of light source 20 will be exemplified. In a first example, the half-value angle is included in a range from ±5° to ±80° inclusive. In a second example, the half-value angle is included in a range from ±9° to ±70°.

The control of light source 20 by controller 30 will be described. Controller 30 controls a predetermined control factor related to light source 20. In one example, the predetermined control factor includes an output of light source 20, an on-duty ratio of light source 20, and a lighting frequency of light source 20.

The output of light source 20 will be exemplified. In one example, the output of light source 20 is included in a range from 30 mW/cm² to 100 mW/cm² inclusive. The on-duty ratio of light source 20 will be exemplified. In a first example, the on-duty ratio is included in a range from 20% to 80% inclusive. In a second example, the on-duty ratio is included in a range from 40% to 80% inclusive. In a third example, the on-duty ratio is included in a range from 40% to 60% inclusive. The lighting frequency of light source 20 will be exemplified. In a first example, the lighting frequency is included in a range from 0.1 kHz to 30 kHZ inclusive. In a second example, the lighting frequency is included in a range from 1 kHz to 30 kHz inclusive.

Photocosmetic device 10 may take one or a plurality of modes specified depending on the features of light source 20 and the predetermined control factor. The features of light source 20 include, for example, the number of peaks of light source 20, a peak wavelength of light source 20, a spectral half-value width of light source 20, and a half-value angle of light source 20. Examples of the predetermined control factor include the output of light source 20, the on-duty ratio of light source 20, and the lighting frequency of light source 20. Controller 30 controls light source 20 such that the output of light source 20, the on-duty ratio of light source 20, and the lighting frequency of light source 20 are included in the ranges illustrated in the modes.

Photocosmetic device 10 of a first mode includes the following configuration.

The number of peaks is single. The peak wavelength is included in a range from 900 nm to 980 nm inclusive. The half-value spectral width is included in a range from 40 nm to 80 nm inclusive. The half-value angle is included in a range from ±5° to ±80° inclusive. The power is included in a range from 30 mW/cm² to 100 mW/cm² inclusive. The on-duty ratio is included in a range from 40% to 60% inclusive. The lighting frequency is included in a range from 10 kHz to 30 kHZ inclusive.

Photocosmetic device 10 of a second mode includes the following configuration.

The number of peaks is single. The peak wavelength is included in a range from 900 nm to 980 nm inclusive. The half-value spectral width is included in a range from 40 nm to 80 nm inclusive. The half-value angle is included in a range from ±5° to ±80° inclusive. The power is included in a range from 30 mW/cm² to 100 mW/cm² inclusive. The on-duty ratio is included in a range from 40% to 80% inclusive. The lighting frequency is included in a range from 10 kHz to 30 kHZ inclusive.

Photocosmetic device 10 of a third mode includes the following configuration.

The number of peaks is single. The peak wavelength is included in a range from 900 nm to 980 nm inclusive. The half-value spectral width is included in a range from 40 nm to 80 nm inclusive. The half-value angle is included in a range from ±5° to ±80° inclusive. The power is included in a range from 30 mW/cm² to 100 mW/cm² inclusive. The on-duty ratio is included in a range from 40% to 60% inclusive. The lighting frequency is included in a range from 0.1 kHz to 30 kHZ inclusive.

Photocosmetic device 10 of a fourth mode includes the following configuration.

The number of peaks is single. The peak wavelength is included in a range from 900 nm to 980 nm inclusive. The half-value spectral width is included in a range from 40 nm to 80 nm inclusive. The half-value angle is included in a range from ±5° to ±80° inclusive. The power is included in a range from 30 mW/cm² to 100 mW/cm² inclusive. The on-duty ratio is included in a range from 40% to 80% inclusive. The lighting frequency is included in a range from 0.1 kHz to 30 kHZ inclusive.

Photocosmetic device 10 of a fifth mode includes the following configuration.

The number of peaks is single. The peak wavelength is included in a range from 820 nm to 980 nm inclusive. The half-value spectral width is included in a range from 40 nm to 80 nm inclusive. The half-value angle is included in a range from ±5° to ±80° inclusive. The power is included in a range from 30 mW/cm² to 100 mW/cm² inclusive. The on-duty ratio is included in a range from 40% to 80% inclusive. The lighting frequency is included in a range from 0.1 kHz to 30 kHZ inclusive.

Photocosmetic device 10 of a sixth mode includes the following configuration.

The number of peaks is single. The peak wavelength is included in a range from 900 nm to 980 nm inclusive. The half-value spectral width is included in a range from 40 nm to 80 nm inclusive. The half-value angle is included in a range from ±5° to ±80° inclusive. The power is included in a range from 30 mW/cm² to 100 mW/cm² inclusive. The on-duty ratio is included in a range from 20% to 80% inclusive. The lighting frequency is included in a range from 0.1 kHz to 30 kHZ inclusive.

Photocosmetic device 10 of a seventh mode includes the following configuration.

The number of peaks is single. The peak wavelength is included in a range from 600 nm to 980 nm inclusive. The half-value spectral width is included in a range from 40 nm to 80 nm inclusive. The half-value angle is included in a range from ±5° to ±80° inclusive. The power is included in a range from 30 mW/cm² to 100 mW/cm² inclusive. The on-duty ratio is included in a range from 40% to 80% inclusive. The lighting frequency is included in a range from 0.1 kHz to 30 kHZ inclusive.

Photocosmetic device 10 according to an eighth mode includes the following configuration.

The number of peaks is single. The peak wavelength is included in a range from 600 nm to 980 nm inclusive. The half-value spectral width is included in a range from 40 nm to 80 nm inclusive. The half-value angle is included in a range from ±5° to ±80° inclusive. The power is included in a range from 30 mW/cm² to 100 mW/cm² inclusive. The on-duty ratio is included in a range from 20% to 80% inclusive. The lighting frequency is included in a range from 0.1 kHz to 30 kHZ inclusive.

### (Examples)

A test for confirming a cosmetic effect by the photocosmetic device will be described. A subject is a three-dimensional cultured skin model. The three-dimensional cultured skin model has a layer of cells corresponding to a basal cell layer, a spinous cell layer, a granular cell layer, and a horny layer. Examples of the three-dimensional cultured skin model used in the test include a cultured model and a commercially available product. Examples of the three-dimensional cultured skin model of KURABO INDUSTRIES LTD. include EPI-200, EPI-200X, EPI-606, and EPI-606X. Examples of the three-dimensional cultured skin model of TOYOBO CO., LTD. include TESTSKINTMLSE, TESTSKINTMLSE-d, and TESTSKINLSE-high. EPI-200 was selected as the three-dimensional culture model of the subject.

A preparation process before illumination light illuminated the subject was performed in the following procedure. The subject was set on a maintenance medium in an incubator and was cultured under predetermined culture conditions for a predetermined culture time. As the predetermined culture conditions, a temperature in a tank of about 37°C, a humidity in the tank of about 95%, and a CO₂ concentration of about 5% were selected. About 24 hours was selected as the predetermined culture time.

A chemical substance having a damaging action on the skin was mixed into a solvent to form a solution. A concentration of the chemical substance was adjusted to a predetermined concentration. In a preferred example, the predetermined concentration is determined such that there is no or substantially no action of the chemical substance under invivo conditions. The subject was immersed in the solution for a predetermined time. In a preferred example, the predetermined time is determined such that there is substantially no action of the solution on the subject and the solution is sufficiently in contact with the cells of the subject. Sodium lauryl sulfate was selected as the chemical substance. 5% by mass was selected as the predetermined concentration. 15 minutes was selected as the predetermined time.

The immersed subject was washed with 10 ml of phosphate buffered saline. The washed subject was transferred from the incubator to a clean bench. After the transfer of the subject, the subject was allowed to stand until a predetermined waiting time had elapsed. 20 minutes was selected as the predetermined waiting time. After the lapse of the predetermined waiting time, illumination light illuminated the subject by using a photocosmetic device. The number of times of illumination of the illumination light is once a day. An illumination period is 8 days. The number of times of illumination during the illumination period is eight in total.

In addition to the subject irradiated by the illumination light (hereinafter, referred to as a "first-type subject"), a subject not irradiated with the illumination light (hereinafter, referred to as a "second-type subject") was prepared. Conditions for preparing the second-type subject are the same as the conditions for the first-type subject except that the illumination light is not illuminated. A predetermined number of first-type subjects and second-type subjects were prepared. 5 was selected as the predetermined number.

Transepidermal water loss (hereinafter, referred to as "TEWL") and a damage level were selected as items for confirming the cosmetic effect of the photocosmetic device. The TEWL and the damage level were measured for the first-type subjects and the second-type subjects. A device used for the measurement of the TEWL is VAPO SCAN (manufactured by Asahi Techno Lab. Ltd.). The damage level was measured by visual measurement by an examiner.

A TEWL recovery rate was obtained from the measurement result of the TEWL. The TEWL recovery rate is a ratio of the TEWL of the first-type subject to the TEWL of the second-type subject. Four levels of A to D were set for the TEWL recovery rate. A is a case where the TEWL recovery rate is less than 0.7. B is a case where the TEWL recovery rate is equal to or greater than 0.7 and is less than 0.85. C is a case where the TEWL recovery rate is equal to or greater than 0.85 and is less than 1.0. D is a case where the TEWL recovery rate is equal to or greater than 1.0. Two levels of A and B were set as the damage level. A is a case where the damage to the subject is not confirmed. B is a case where a slight burn injury is confirmed in the subject.

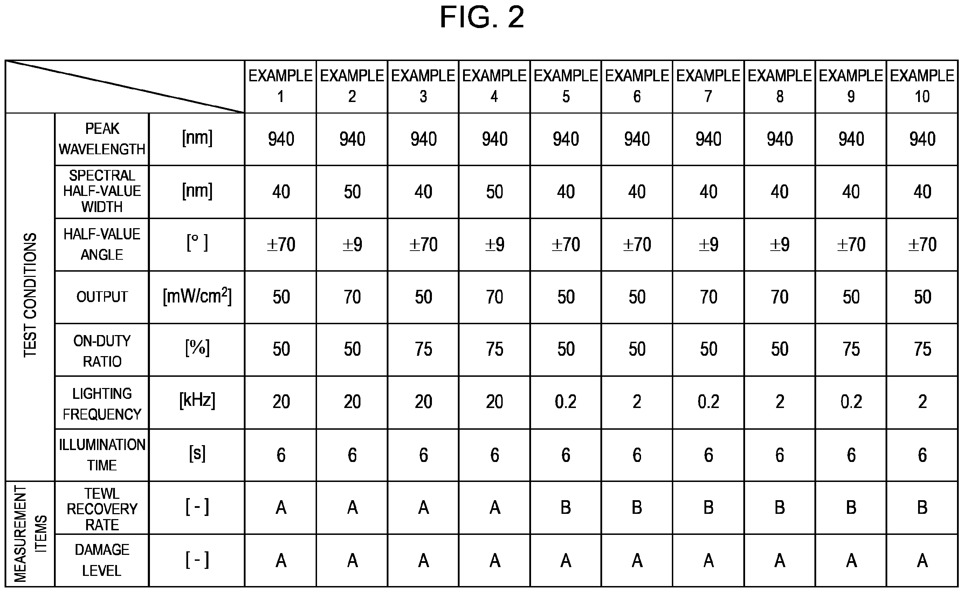

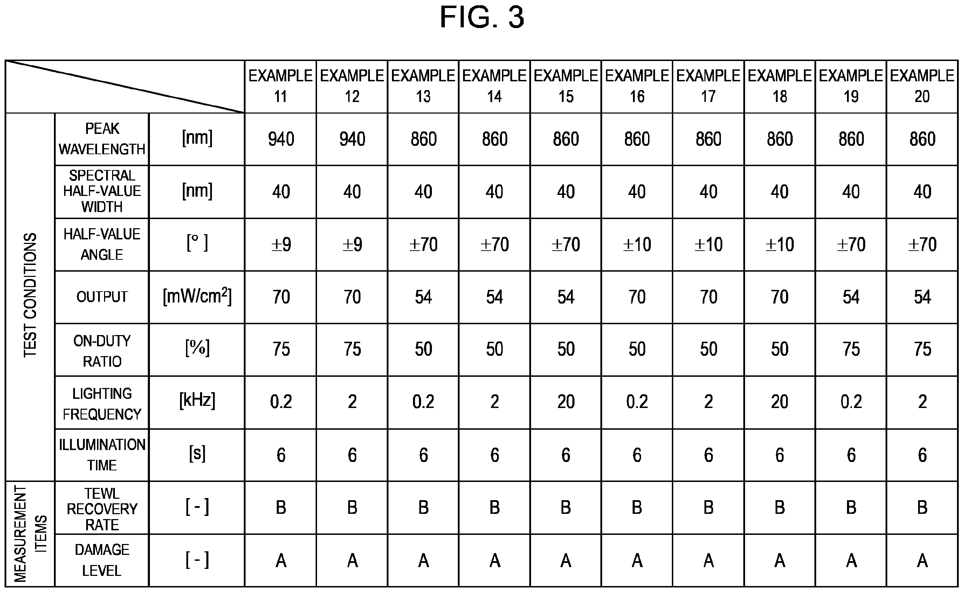

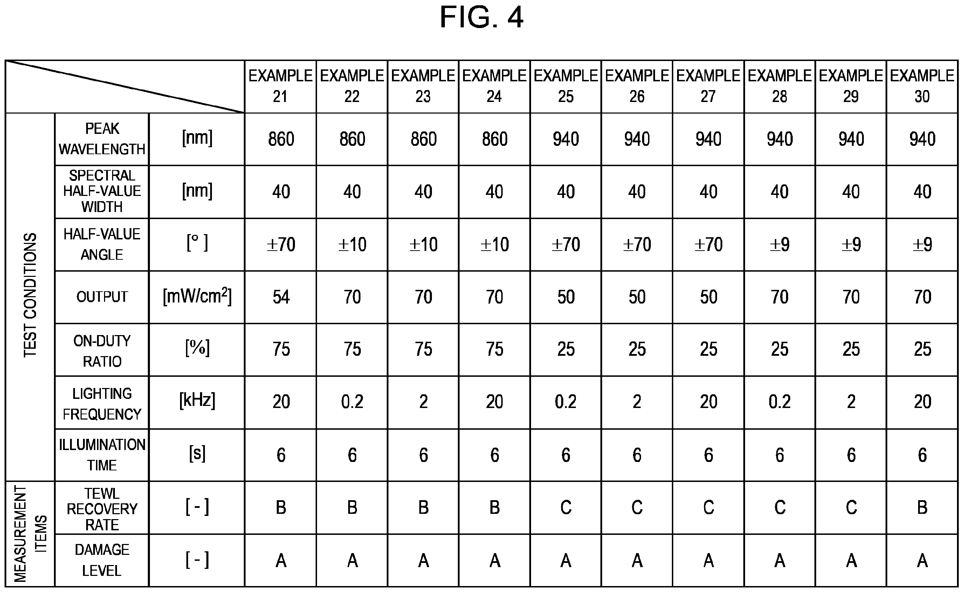

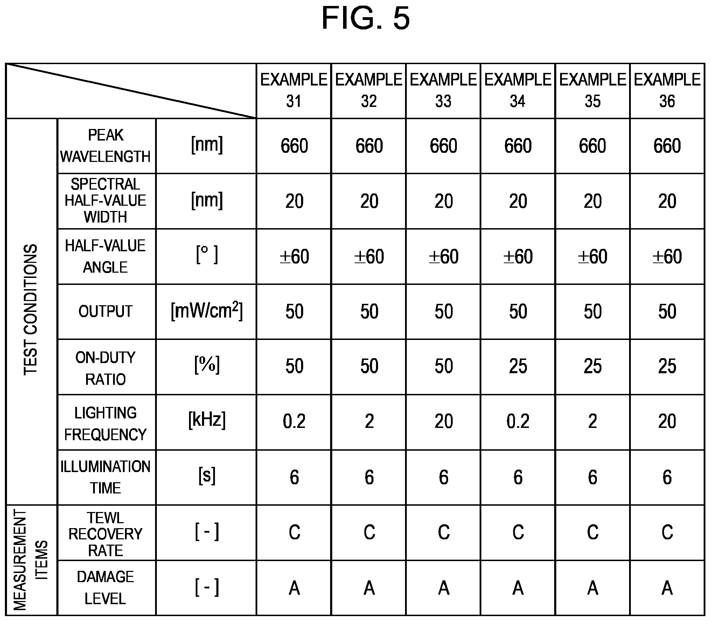

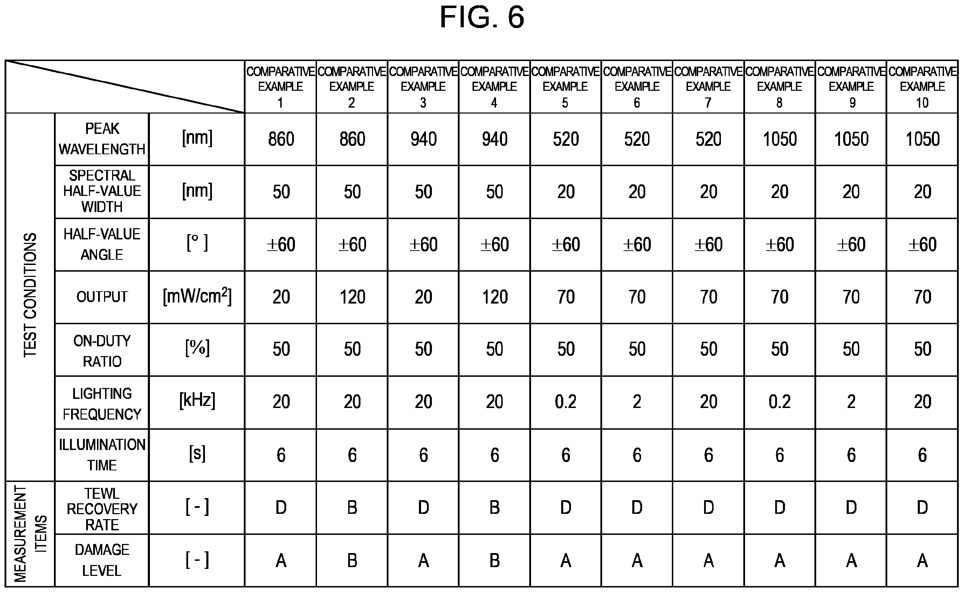

The above test was performed on Examples 1 to 36 and Comparative Examples 1 to 10 illustrated in Figs. 2 to 6. In the individual Examples and Comparative Examples, at least one of the following test conditions is set to a condition different from the conditions of the other Examples and Comparative Examples. The test conditions are a peak wavelength, a spectral half-value width, a half-value angle, an output, an on-duty ratio, a lighting frequency, an illumination time, and total energy. The number of peaks in each of Examples and Comparative Examples is single. In Examples, it was confirmed that a preferable effect was obtained as compared with Comparative Examples.

The description of the above exemplary embodiment and examples is not intended to limit modes that can be taken by the photocosmetic device according to the present disclosure. The photocosmetic device according to the present disclosure can take a mode different from the modes exemplified in the exemplary embodiment and examples. One example thereof is a mode in which a part of the configurations of the exemplary embodiment and examples is replaced, changed, or omitted, or a form in which a new configuration is added to each of the exemplary embodiment and examples.

The photocosmetic device of the present disclosure can be used for skin beauty and the like.

### REFERENCE MARKS IN THE DRAWINGS

- 10: photocosmetic device
- 20: light source
- 30: controller

## Claims

1. A photocosmetic device comprising:
a light source that outputs illumination light that illuminates a target portion; and
a controller that controls an output of the light source, a lighting frequency of the light source, and an on-duty ratio of the light source,
wherein a peak wavelength of the light source is included in a range from 600 nm to 980 nm inclusive,
a spectral half-value width of the light source is included in a range from 20 nm to 80 nm inclusive, and
the controller controls the light source, the output of the light source being included in a range from 30 mW/cm² to 100 mW/cm² inclusive, the lighting frequency of the light source being included in a range from 0.1 kHz to 30 kHz inclusive, and the on-duty ratio of the light source being included in a range from 20% to 80% inclusive.

2. The photocosmetic device according to Claim 1, wherein the on-duty ratio of the light source is included in a range from 40% to 80% inclusive.

3. The photocosmetic device according to Claim 2, wherein the on-duty ratio of the light source is included in a range from 40% to 60% inclusive.

4. The photocosmetic device according to any one of Claims 1 to 3, wherein the peak wavelength of the light source is included in a range from 820 nm to 980 nm inclusive.

5. The photocosmetic device according to Claim 4, wherein the peak wavelength of the light source is included in a range from 900 nm to 980 nm inclusive.

6. The photocosmetic device according to any one of Claims 1 to 5, wherein the lighting frequency of the light source is included in a range from 10 kHz to 30 kHz inclusive.
